# EUROPEAN PATENT APPLICATION

(11) **EP 2 006 685 A1**
(43) Date of publication of application: **24.12.2008**
(21) Application number: 07740536.3
(22) Date of filing: 30.03.2007
(51) Int. Cl.: G01N 33/543, C07K 16/18, C12N 5/10, C12P 21/08

(54) **IMMUNE AGGLUTINATION REACTION REAGENT KIT AND METHOD OF ASSAYING ANTIGEN**

(30) Priority: 31.03.2006 JP 2006100871
(71) Applicant: NISSUI PHARMACEUTICAL CO., LTD., Taito-ku, Tokyo 110-8736 (JP)
(72) Inventor: NATSUBAYASHI, Tadashi, Yuuki-shi Ibaraki 307-0036 (JP); TERUNUMA, Ikuo, Yuuki-shi Ibaraki 307-0036 (JP); TAKANO, Akinori, Yuuki-shi Ibaraki 307-0036 (JP)
(74) Representative: Fiener, Josef
(86) International application number: PCT/JP2007/057100
(87) International publication number: WO 2007/114337

(57) **Abstract**

It is intended to provide a method by which an antigen in a specimen can be directly assayed without pretreating the specimen in the case where there are only a small.number of epitopes or there is a different epitope located close and thus the reaction with an antibody is carried out only at a low efficiency due to a steric hindrance or the like. Different latexes are sensitized respectively with two kinds of monoclonal antibodies recognizing different epitopes. After reacting a specimen with a latex reagent having been sensitized with one monoclonal antibody, the resultant liquid reaction mixture is further reacted with the latex reagent having been sensitized with the other monoclonal antibody. Thus, an antigen can be directly, highly efficiently and highly sensitively assayed without pretreating the specimen.

SEQUENCE LISTING

<110> Nissui Pharmaceutical CO., LTD.

<120> Kit for immune agglutination reagent and assay of antigen

<130> NSM0055

<160> 6

<170> PatentIn version 3.1

<210> 1
<211> 12
<212> PRT
<213> Artificial

<220>
<223> Antigen recognition site in a conventional antibody

<400> 1

Val Leu Ala Glu Asn Tyr Lys Ser Asp Asn Cys Glu
1               5                   10

<210> 2
<211> 13
<212> PRT
<213> Artificial

<220>
<223> Antigen recognition site in a conventional antibody

<400> 2

Gln His Leu Phe Gly Ser Asn Val Thr Asp Cys Ser Gly
1               5                   10

<210> 3
<211> 17
<212> PRT
<213> Artificial

<220>
<223> Antigen recognition site in a conventional antibody

<400> 3

Val Val Ala Arg Ser Met Gly Gly Lys Glu Asp Leu Ile Trp Glu Leu
1               5                   10                  15

Leu

<210> 4
<211> 14
<212> PRT
<213> Artificial

<220>

<223>  Antigen recognition site in a conventional antibody

<400>  4

Ile Ala Lys Ile Met Asn Gly Glu Ala Asp Ala Met Ser Leu
1               5                   10


<210>  5
<211>  13
<212>  PRT
<213>  Artificial

<220>
<223>  Antigen recognition site in a conventional antibody

<400>  5

Tyr Glu Lys Tyr Leu Gly Glu Glu Tyr Val Lys Ala Val
1               5                   10


<210>  6
<211>  15
<212>  PRT
<213>  Artificial

<220>
<223>  Antigen recognition site in a conventional antibody

<400>  6

Ser Lys Leu Ser Met Gly Ser Gly Leu Asn Leu Ser Glu Pro Asn
1               5                   10                  15

## Description

### TECHNICAL FIELD

The invention incorporates two types of monoclonal antibodies that recognize different epitopes. It pertains to an immune agglutination reagent kit and method of measuring antigen that enables the accurate measurement of a specific antigen in a specimen by a simple process.

### BACKGROUND ART

Immunoassay method using antigen-antibody reaction has an important role in the clinical diagnosis of various endocrine diseases. To date, a wide variety of immunoassay methods have been known.

Among them, there is the method of measuring antigen in a specimen using an immune agglutination reagent formed by antibodies sensitized on carrier particles. The immune agglutination reagent is made to react with the antigen in the test substance.
After adding the reagent to the agglutination formed as a result of this antigen antibody reaction, the amount of change in absorbance is measured and compared with that of a known standard serum concentration to determine the final antigen concentration.

When carrier particles such as latex are used for sensitization, the individual antibodies are sensitized by the different carrier particles. The various sensitized carrier particles are then mixed together in just proportions to form an immune agglutination reagent. (Patent Document 1: Japanese Patent Publication No. 3-40341)

However, in using the above-mentioned mixed immune agglutination reagent, when the number of epitopes in the antigen was low or the reaction efficiency with the antibody was poor due to steric hindrance caused by various epitopes being close to each other in the antigen, the amount of latex aggregates formed was inadequate, reproducibility was poor because the desired absorbance was not achieved, and the accurate measurement was ultimately not possible.

In such a case, a measuring reagent is sometimes provided based on the reaction principle of the competition technique. However, the range of measurement of the competition technique is generally narrow with reproducibility of the low concentration end and high concentration end being poor. The reagent is, therefore, not suitable for accurate measurement.

On the other hand, a method of identifying alcoholics by measuring carbohydrate (sugar chain) deficient transferrin (CDT) levels has been gaining some attention. Asialo-, monosialo- and disialo-transferrin are called carbohydrate (sugar chain) deficient transferrin (CDT). CDT has been found to be a significant marker for detecting and monitoring alcohol consumption especially chronic alcohol consumption.

Immunological test method of CDT has been reported in WO96/26444 (Patent Document 2) and Heil et al., Anaesthesist, 43, 447-453, (1994) (Non-Patent Document 1). In this method, a diluted serum sample is passed through an anion-exchange resin wherein normal transferrin isotopes are retained in the resin while allowing CDT fractionation to pass through. CDT composition of this eluate is measured by immunoturbidimetric assay. Other methods such as HPLC method in which CDT is assayed are reported in WO95/04932 (Patent Document 3).

All of the above-mentioned methods traditionally used in testing alcoholics involve ion-exchange resin or HPLC deeming them to be relatively complicated.

Makhlouf et al. tried to form a monoclonal antibody against CDT in order to directly measure CDT alone. They manufactured an antibody in which the immunogen is a synthetic peptide composed of 6 amino acids in length forward and backward of Asn 413 and Asn 611 in which sugar chains of transferrin are bound. Their plan was to use this antibody in the immunoassay method of measuring CDT (Patent Document 4: WO93/06133).

In turn, ER.Trimble et al. reported that the method used by Makhlouf et al. using manufactured monoclonal antibody with synthesized peptide as an antigen did not react with CDT because the epitope is concealed within the transferrin molecule and therefore cannot be used for detection (Non-Patent Document 2: Biochem Soc Trans, 26 (1), S48 (1998)).

In the Japanese Patent Laid-Open No. 2004-051633 (Patent Document 5), an antibody that does not require CDT to be bound in solid phase is described because it is selectively bound to CDT in an aqueous solution. This antibody is provided by using recombinant nonglycosylated transferrin as immunogen obtained via gene recombination procedure, or it is provided by using a non-glycosylated transferrin as an immunogen obtained by deglycosylation reaction with enzymes. In other words, it is a man-made antibody provided by using CDT as an immunogen.

CDT measuring regents currently distributed on the market use rabbit monoclonal antibody for the column method. Iron saturation of the specimen as well as separation of CDT at the column is thus necessary for CDT analysis, making the procedure cumbersome. In addition, CDT measurement in the HPLC method involves a long period of time per specimen and thus is unsuitable for processing multiple specimens.

Recently, reagents for CDT (carbohydrate deficient transferring) analysis that do not require pretreatment of specimen have become available (Non-Patent Document 2: D. Kraul et al., Clinical Chemistry, vol. 51, No. 6 96 B-149 (2005) (2005 AACC Annual meeting summary). However, the measurement principle is based on the competition technique with the range of measurement being narrow causing poor reproducibility of the low concentration end and high concentration end. The reagent is, therefore, not suitable for accurate measurement.

Moreover, since the above CDT measuring reagent is exclusive, analytical equipment that can be used is limited, and use of a general-purpose automated analyzer is difficult.

Patent Document 1: Japanese Patent Publication No. 3-40341
Patent Document 2: WO96/26444
Patent Document 3: WO95/04932
Patent Document 4: WO93/06133
Patent Document 5: Japanese Patent Laid-Open No. 2004-051633
Non-Patent Document 1: Heil et al., Anaesthesist, 43, 447-453, (1994)
Non-Patent Document 2: Biochem Soc Trans, 26(1), S48 (1998)
Non-Patent Document 3: D. Kraul et al., Clinical Chemistry, vol. 51, No. 6 96 B-149(2005) (2005 AACC Annual Meeting Summary)

### DISCLOSURE OF INVENTION

### Problem to be Solved by the invention:

After dedicating themselves to research, the inventors were able to prepare CDT that is native and natural and to obtain several types of anti-CDT monoclonal antibody that recognizes the CDT structure. However, they realized that agglutination could not be achieved well when conventional immune agglutination reagent described above was used for measurement. In other words, various carrier particles were sensitized by the respective anti-CDT monoclonal antibody and then mixed together in just proportions to form an immune agglutination reagent. Although this reagent was used, immune agglutination did not go well against the specimen.

The objective of this invention is to provide a method for measuring antigen based on immune agglutination in order to improve the accuracy of the measurement and to expand the range of measurement. Pretreatment of the specimen is not required and general-purpose automated analytical equipment can be used thus cutting down on economical costs. The objective of this invention is also to provide an immune agglutination reagent kit for use in the method of measuring. In addition to these objectives, this invention provides an immune agglutination reagent kit for measuring the CDT amount within a specimen using efficient immune agglutination, anti-CDT monoclonal antibody for use in the immune agglutination reagent kit, and method for measuring CDT using the immune agglutination reagent kit.

The antibody used in the immune agglutination reagent kit of this invention should ideally be one that does not require pretreatment of CDT within the biological fluid. Antibody that recognizes the natural CDT structure must be obtained.

On the other hand, as reported by ER. Trimble et al. (Non-Patent Document 7), the area near the sugar chain deficient region may be concealed within the CDT molecule. Several disulfide bonds within the transferrin molecule make up a strong conformation, and it is presumably difficult to obtain an antibody that would recognize epitopes near the sugar chain deficient region.

In addition, in terms of immunogen, there is a great difference between artificially manufactured CDT and natural CDT. For example, with the gene recombinant mentioned in Patent Document 4, protein conformation tends to be incomplete because of disulfide binding, and with deglycosylated substance, denaturalization treatment must be done during adjustments. In addition, displacement of amino acid occurs in gene recombinant and deglycosylated substance.

As a purification method of natural CDT from biological fluid, affinity chromatography is generally used where anti-transferrin antibodies are bound to a column. Transferrin is made to bind to anti-transferrin antibodies in the column after which the antigen-antibody complex is allowed to denaturalize under acidic condition. Purification is done by eluting transferrin containing CDT. However, since there is a high possibility that CDT obtained by this method is denatured, the immunogen is unsuitable.

Based on the existing problems, artificially manufactured CDT as immunogen was deemed unsuitable because the antibody that may not respond to CDT in biological fluid may be produced. The purpose of this invention is to provide suitable anti-CDT antibody that can selectively bind to natural CDT and can be used for measurement based on immune agglutination.

### Solution to the Problems:

The immune agglutination reagent kit invented for the purpose of solving the above mentioned problems is characterized by two types of sensitized carrier particles that are independently preserved. The immune agglutination reagent contains two types of carrier particles produced by separate sensitization with two types of monoclonal antibodies that recognize different epitopes. When the two types of sensitized carrier particles are made to react simultaneously with a specimen containing the target antigen, the agglutination reaction is weak. The agglutination reaction intensifies when the order of reaction of sensitized carrier particles is specified for a specific type of monoclonal antibody.

Method for measuring antigen of the invention is characterized by preparing the above-mentioned immune agglutination reagent kit. After using one type of reagent against the specimen suspected to contain antigen, the reaction solution is made to react with the other type of reagent to cause agglutination. The amount of change in agglutination can thus be measured.

Although monoclonal antibody used in the immune agglutination reagent kit of the invention may be derived from mice, more than two kinds of monoclonal antibodies that recognize different epitopes may be used regardless of animal type.

More than two kinds of monoclonal antibodies that recognize different epitopes may be used. In one reagent, carrier particles sensitized with more than two kinds of monoclonal antibodies or a mixture of two types of carrier particles separately sensitized with two types of monoclonal antibodies with different epitopes may be used. In the other reagent, carrier particles sensitized with a monoclonal antibody recognizing yet another epitope, may be used.

The preferred antigen in this invention is CDT. The particularly preferred immune agglutination reagent kit used for measuring CDT is described as follows: the first reagent made to react contains carrier particles sensitized with anti-CDT mouse monoclonal antibodies produced by Hybridoma NCDT503 deposited at International Patent Organism Depositary (IPOD) (Date received: Mar. 14, 2006; Registration no.: FERM P-20844), and the second reagent contains carrier particles sensitized with anti-CDT mouse monoclonal antibodies produced by Hybridoma NCDT077 deposited at International Patent Organism Depositary (IPOD) (Date received: Mar. 14, 2006; Registration no.: FERM P-20843).

Method for measuring antigen is characterized as follows. After using reagent containing carrier particles sensitized with anti-CDT mouse monoclonal antibodies produced by Hybridoma NCDT503 (FERM P-20844) against the specimen suspected to contain CDT, the reaction solution is made to react with reagent containing carrier particles sensitized with anti-CDT mouse monoclonal antibodies produced by Hybridoma NCDT077 (FERM P-20843) to generate agglutination. The amount of change in agglutination can thus be measured. The pH of the above mentioned reaction solution should be more than 6.8 and less than 8.5.

In the method of measuring the antigen of this invention, either an automated analyzer or a manual method may be used. For general-purpose automated analyzers, if it is analytical equipment capable of handling analysis involving a series of three reagents, it can be used for the reagent kit in question. An automated analyzer of the 7070 model or 7170 model etc. made by Hitachi, for example, can be used in three reagents analysis.

Any insoluble carrier particle ordinarily used in immune agglutination reagents would be suitable in the reagents constituting the immune agglutination reagent kit of this invention. Examples are but not restricted to: polystyrene, styrene-methacrylic acid copolymer, styrene-glycidyl methacrylate copolymer, styrene-styrenesulfonate copolymer, methacrylic acid polymer, acrylic acid polymer, acrylonitrile butadiene styrene copolymer, vinyl chloride-acrylic acid ester copolymer, polyvinyl acetate acrylate, and other micro particles of organic, high-molecular substance such as latex, latex administered with treatment for physical absorption, or chemical binding mediated by the carboxyl group, an inorganic oxide such as silica, silica-alumina and alumina, inorganic particles incorporating a functional group by administering silane coupling treatment to inorganic oxides, metallic colloid particles, Type 0-human hemocyte, animal derived particles, such as sheep erythrocyte, liposome. In particular, latex particles are widely used because they are synthetic carriers, their surface can be chemically treated for any purpose, and non-specific does not easily occur. Various kinds of denatured latex and magnetic latex may also be used as needed.

There is no special requirement for the particle size of the above carrier. However, when considering ease of agglutination occurring after an antigen-antibody reaction and ease of discrimination of agglutination, the average particle size should be more than 0.05 µm and less than 1 µm, the more preferred particle size should be more than 0.1 µm and less than 0.5 µm, the most preferred particle size should be more than 0.1 µm and less than 0.2 µm.

The amount used for the carrier can be determined from time to time based on type of antibody or antigen. However, from the perspective of supported efficiency and operability, the carrier particle concentration in the solution at the time of measurement should be more than 0.02% w/w and less than 0.25% w/w, the more preferred particle concentration should be more than 0.02% w/w and less than 0.17% w/w, the most preferred particle concentration should be more than 0.04% w/w and less than 0.13% w/w. The concentration of monoclonal antibodies used in this invention should be more than 0.03 mg/mL and less than 0.09 mg/mL, the more preferred concentration of monoclonal antibodies should be more than 0.03 mg/mL and less than 0.07 mg/mL, the most preferred concentration of monoclonal antibodies should be more than 0.05 mg/mL and less than 0.07 mg/mL.

The buffer solution used in the analysis may be any buffer solution with pH range 6.8 to 8.5, such as Tris, BES, MOPS, TES, HEPES, DIPSO, TAPSO, POPSO, HEPPSO, EPPS, Tricine, Bicine, as well as TAPS.

### Benefits of Invention:

In the conventional method, carrier particles are separately sensitized by a type of antibody that recognizes different epitopes, and these various sensitized carrier particles are then mixed together in just proportions to form an immune agglutination reagent. In allowing agglutination to occur with this reagent, the amount of latex aggregates formed may be inadequate due to the number of epitopes in the antigen being low or due to steric hindrance caused by various epitopes being close to each other. As a result, the desired absorbance may not be achieved. When the kit in the invention containing two types of sensitized carrier particles that are independently preserved is used and one type of reagent is made to react with the specimen suspected to contain antigen and then the other type of reagent is made to react with the reaction solution, agglutination is formed and a substantial change in absorbance can be attained. Measuring antigen in the specimen is thus made possible.

### Brief Description of Drawings:

Fig. 1: A graph of the CDT fraction elution profile by anion-exchange chromatography.
Fig. 2: Photos of SDS-PAGE as well as isoelectric focusing for each fraction of the elution profile in Fig. 1.
Fig. 3: A graph describing the correlation test by sandwich ELISA combined with anti-transferrin polyclonal antibody
Fig. 4: A graph showing the relationship of absorbance with CDT concentration in the following conditions: A) Mixing 2 types of latex reagents, B) Allowing Latex reagent 1 to react first followed by Latex reagent 2, C) Allowing Latex reagent 2 to react first followed by Latex reagent 1.
Fig. 5: A graph showing the relationship of absorbance with CDT concentration when particle size of latex particles are changed.
Fig. 6: A graph showing the relationship of absorbance with CDT concentration when latex concentration in the reaction solution is changed.
Fig. 7: A graph showing the relationship of absorbance with CDT concentration when monoclonal antibody concentration in the reaction solution is changed.
Fig. 8: A graph showing the relationship of absorbance with CDT concentration in relation to usable pH range.
Fig. 9: A graph showing the relationship of absorbance with CDT concentration in relation to results from measuring antigen overage up to 120 mg/dl from purified CDT fraction.

### Working Examples:

This invention is explained further in detailed and practical terms through the following working examples. However, working examples of the invention are not limited to the following.
The following working example involves two types of anti-CDT mouse monoclonal antibodies that recognize different epitopes that are separately sensitized on latex. Immunoassay is carried out by formulating three series of CDT measuring reagents that can be used with general-purpose automated analyzers for which pretreatment of specimen, such as column preparation, is not required.

### Example 1: Preparation of a native and natural CDT fraction

An iron saturated solution (250 µl of 0.5M NaHCO₃ and 180 µl of 10mM FeCl₃) was added to 10 mL of a high-level CDT human serum and incubated for an hour at 4°C. A delipidated solution (100µl of 100 g/l sulfuric dextran and 500 µl of 1M CaCl₂) was then added and the supernatant of the solution was collected. In addition, the albumin was removed using Blue Sepharose column (Product name: Blue Sepharose 6 Fast Flow; manufactured by GE Healthcare) followed by the removal of globulin using a Protein G column (Product name: Hi Trap Protein G HP, manufactured by GE Healthcare) to ultimately remove all proteins besides transferrin. CDT fraction containing native disialo-transferrin and asialo transferrin was prepared by using an anion-exchange column (Product name: Mono Q HR; manufactured by GE Healthcare).

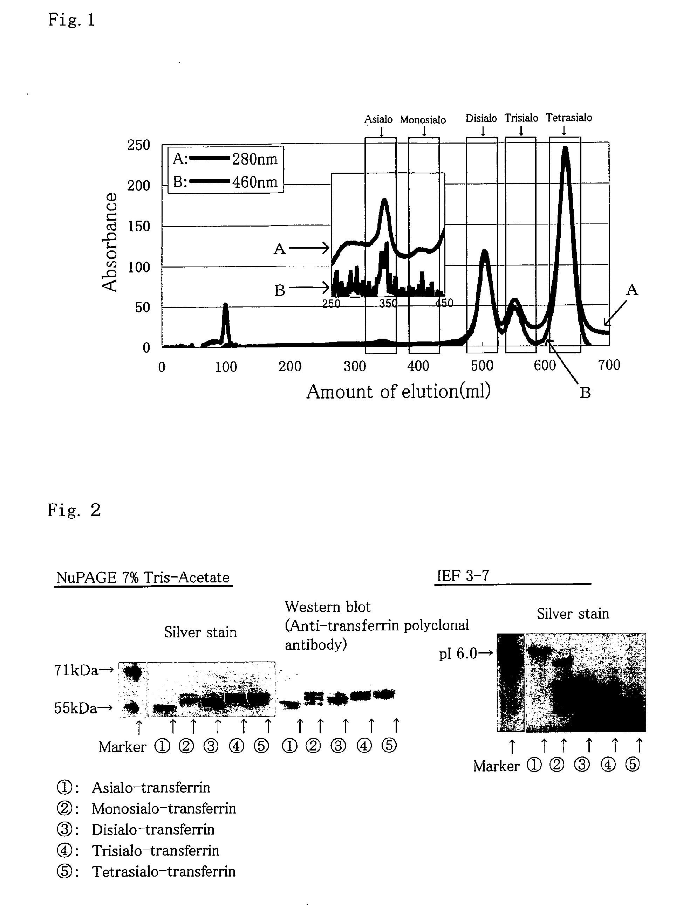

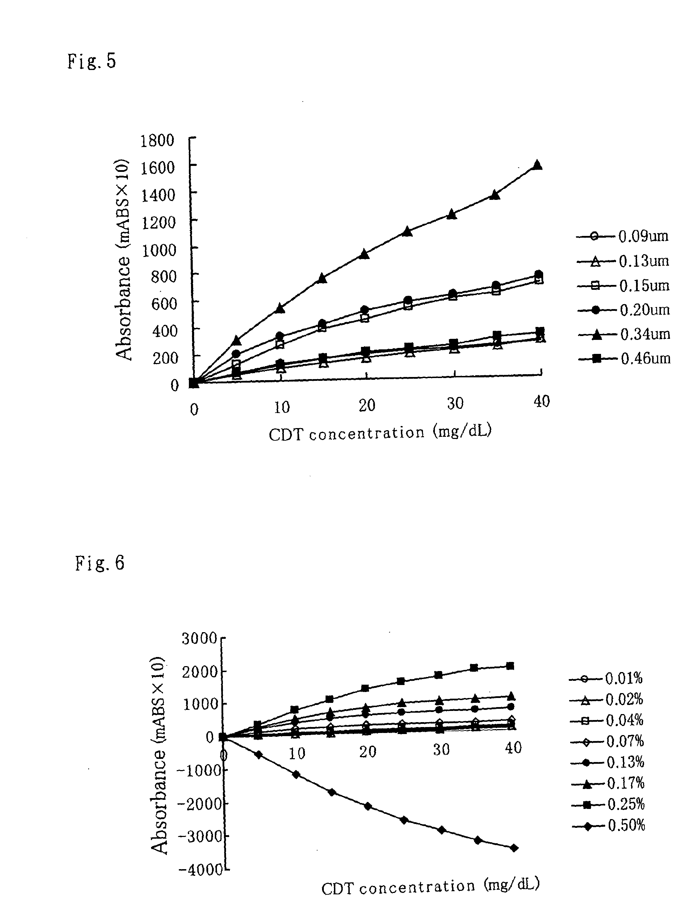

Fig. 1 shows a graph of the CDT fraction elution profile by anion-exchange chromatography. Arrow A in the Drawing shows the absorbance curve at the 280 nm wavelength. Arrow B shows the absorbance curve at the 460 nm wavelength. Fig. 2 shows photos of a silver stain and Western blot for SDS-PAGE (Product name: NuPAGE gel; manufactured by Introvitrogen Corp.) as well as isoelectric focusing for each fraction of the elution profile in Fig. 1. As shown in Fig. 2, 5 types of isoforms (tetrasialo-, trisialo-, disialo-, monosialo-, asialo-transferrin) complying with the amount of transferrin sugar chains were successfully prepared in native and highly purity.

### Example 2: Preparation of hybridoma

The fraction containing disialo- and asialo-transferrin obtained in the above Example 1 was used as a CDT antigen. The antigen solution with a concentration of 0.5 mg/mL was prepared from the CDT antigen. 500µl adjuvant (Product name: TiterMax Gold; manufactured by TiterMax Co.) was mixed in with 500 u l of antigen solution and then emulsified. This was used to subcutaneously immunize a 5-week old BALB/c mouse. Three cycles of booster using the same prepared solution was performed with 2 weeks in between cycles. During this period, a blood sample was taken at the time of immunization to measure antibody activity in the blood. 14 days after the last immunization, 100 µl of antigen solution was administered into the abdominal cavity and 3 days later the spleen was extracted. Splenic cells were made to react for 2 min. in the presence of mouse myeloma cells (P3x63Ag8.653) and polyethylene glycol 4000 (Product name; Merck Inc.) for fusion to occur after which they were suspended in a HAT selective medium, dispensed in a 96-well culture plate and cultivated in a 37 °C CO₂ incubator to prepare hybridoma.

### Example 3: Selection of antibody producing hybridoma for CDT fraction

With regards to hybridoma obtained in the above Example 2, selection of antibody producing hybridoma of this invention was done by sandwich ELISA. Anti mouse antibody adjusted to a 10 µg/mL with PBS was added to a 96-well microplate, 50 µI per well, and made to react all day and night at 4°C. The wells were then washed once with PBS and blocked with 0.5% BSA-PBS to be used as the plate for screening. 50µl of culture supernatant from wells with confirmed hybridoma growth was added to the wells and made to react for 1 hour at room temperature. After washing with wash solution (0.05% Tween-PBS), the CDT fraction mentioned above in Example 1 is made to react for 1 hour at room temperature and similarly washed. A 50 µl alkaline phosphatase labeled anti-transferrin polyclonal antibody is then added and made to react for 1 hour at room temperature. The alkaline phosphatase labeled anti-transferrin polyclonal antibody used was prepared by binding an anti-transferrin polyclonal antibody (manufactured by Shibayagi Co., Ltd.) to an alkaline phosphatase (manufactured by Hoffmann La Roche Ltd.) using the periodate method. After washing again, the coloration of alkaline phosphatase activity was generated using the Kind-King method. The absorbance at 490nm was measured with a microplate reader and monoclonal antibody producing hybridomas, NCDT077 and NCDT503, having antibody activity against the CDT fraction, was selected.

### Example 4: Cloning of monoclonal antibody producing hybridomas against CDT fraction

Cloning of the selected hybridomas in the above Example 3 was done twice by limiting dilution to obtain an established cell line. The obtained Hybridoma NCDT077 was deposited at the International Patent Organism Depositary (IPOD) (Date received: Mar. 14, 2006; Registration no.: FERM P-20843) and Hybridoma NCDT503 was deposited at the International Patent Organism Depositary (IPOD) (Date received: Mar.14, 2006; Registration no.:FERM P-20844).

### Example 5: Preparation of antibody

With regards to each established cell line obtained in the above Example 4, monoclonal antibody producing hybridoma with antibody activity against CDT fraction was made to breed in the mouse abdominal cavity for mass production of monoclonal antibody. Monoclonal antibody in the mouse abdominal fluid thus obtained was purified by column chromatography using a Protein G column (manufactured by GE).

### Example 6: Determination of antibody subclass

A IgG2a (κ) was chosen as the subclass for monoclonal anti-CDT antibody produced by Hybridoma NCDT077 in the above Example 3 using Isostrip (Registered trademark; manufactured by Hoffman La Roche Ltd.), and IgG1 (κ) was similarly chosen as the subclass for monoclonal anti-CDT antibody produced by Hybridoma NCDT503.

### Example No. 7: Confirmation of antibody specificity using sandwich ELISA

With regards to monoclonal antibody (Hybridoma NCDT077 producing antibody and Hybridoma NCDT503 producing antibody) prepared in the above Example 5, confirmation of specificity for transferring isoform prepared in the above Example 1 was done using sandwich ELISA. Antibody prepared to 10µg/mL with PBS was added to a 96-well microplate, 50 µl per well, and made to react all day and night at 4°C. The wells were then washed once with PBS and blocked with 0.5% BSA-PBS. 50µl of alkaline phosphatase prepared in the above Example 1 was then added and made to react for 1 hour at room temperature. After washing with a wash solution(0.05% Tween-PBS), a 50 µl alkaline phosphatase labeled anti-transferrin polyclonal antibody (same as that adjusted in the above Example 3) is then added and made to react for 1 hour at room temperature. After washing again, a coloration of alkaline phosphatase activity was generated using the Kind-King method. The absorbance at 490nm was measured with a microplate reader and the specificity of the antibody confirmed by comparing the intensity of reaction to transferrin isoform. These results are shown below in Table1.

**Table 1:**

| Hybridoma (Antibody) no. | OD at 490nm (Absorbance) | | |
|---|---|---|---|
| | Asialo-transferrin (200ng/mL) | Disialo-transferrin (200ng/mL) | Tetrasialo-transferrin (200ng/mL) |
| NCDT077 | 2.902 | 2.889 | 0.421 |
| NCDT503 | 2.124 | 1.946 | 0.034 |

As shown in Table 1, a Hybridoma NCDT077 producing antibody and a Hybridoma NCDT503 producing antibody reacted specifically with a native CDT fraction (asialo and disialo-transferrin) in the solution, but not with tetrasialo-transferrin, a non-CDT transferrin.

### Example 8: Correlation analysis by sandwich ELISA combined with anti transferrin polyclonal antibody

A total of 32 samples of blood serum from healthy people and alcoholic patients were measured by sandwich ELISA using a monoclonal antibody (Hybridoma NCDT077 producing antibody and a Hybridoma NCDT503 producing antibody) prepared in Example 5. Measurement in the control test was taken using a reagent manufactured by Bio-Rad Laboratories. A method measuring %CDT in transferrin using a column was applied in the control test. The amount of total transferrin before separation and amount of CDT after separation in the column were measured. The CDT concentration and %CDT (CDT proportion against total transferrin) in the specimen were then calculated.

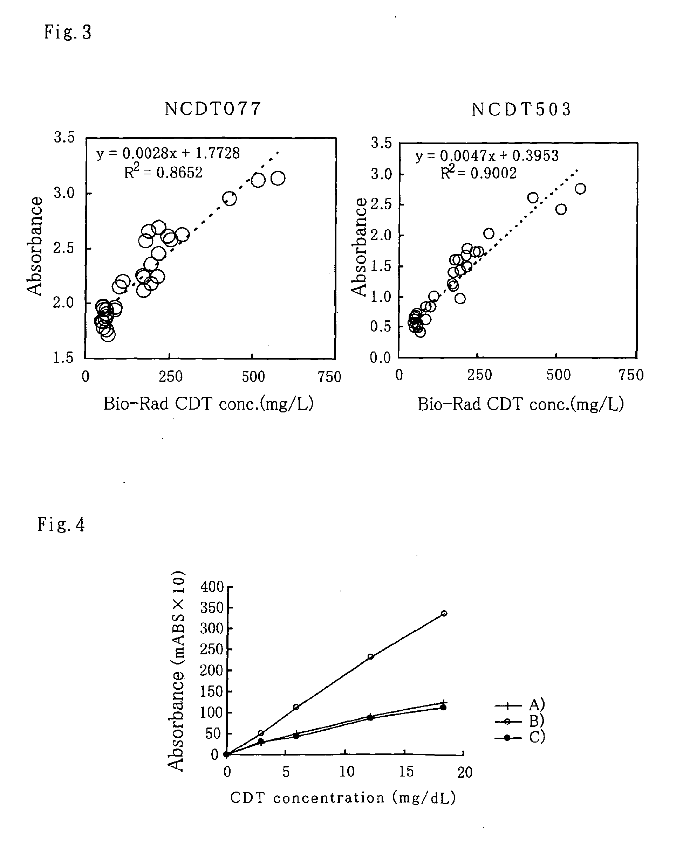

In the above measurement by sandwich ELISA, an antibody adjusted to 10 µg/mL with PBS was added to a 96-well microplate, 50 µI per well, and made to react all day and night at 4°C. The wells were then washed once with PBS and blocked with 0.5% BSA-PBS. 50µl of blood serum from each healthy person and alcoholic patient was added to the wells and made to react for 1 hour at room temperature. After washing with a wash solution (0.05% Tween-PBS), 50 u l alkaline phosphatase labeled an anti-transferrin polyclonal antibody (same as that prepared in the above Example 3) is then added and made to react for 1 hour at room temperature. After washing again, a coloration of alkaline phosphatase activity was generated using the Kind-King method. The absorbance at 490nm was measured with a microplate reader and the correlation with the measurements of the control test confirmed. These results are shown in Fig. 3.

As shown in Fig. 3, a Hybridoma NCDT077 producing antibody and a Hybridoma NCDT503 producing antibody obtained by using a native and natural CDT fraction as immunogen showed a good correlation with the CDT concentration of the control test. Therefore, since absorbance in a Hybridoma NCDT077 producing antibody and a Hybridoma NCDT503 producing antibody is correlated to CDT concentration in the specimen, CDT concentration in the specimen can be assayed by measuring the absorbance.

### Example No. 9: Confirmation of antigen recognition site of the antibody

The reactivity of each monoclonal antibody (Hybridoma NCDT077 producing antibody and Hybridoma NCDT503 producing antibody) prepared in the above Example 5 was examined as follows. After binding peptides with 6 types of amino acid sequence (shown below as P1-P6), to ELISA plate, antibody adjusted to 10µg/mL was added to each well, 50 µl per well, and made to react for 1 hour at room temperature. After washing with a wash solution (0.05% Tween-PBS), a 50 µl alkaline phosphatase labeled anti-mouse IgG antibody (Product name: Goat F (ab') Anti-Mouse Ig's, Alkaline Phosphatase Conjugate AMI4405; manufactured by Biosource International) is then added and made to react for 1 hour at room temperature. After washing again, a coloration of alkaline phosphatase activity was generated using the Kind-King method and the absorbance at 490nm measured with a microplate reader.

The underlined section of the peptide sequence P1-P6 indicates the antibody recognition site in Patent Document 3 and Patent Document 4. Results are shown in Table2.
P1 VLAENYNKSDNCE (Sequence No. 1)
P2 QHLFGSNVTDCSG (Sequence No. 2)
P3 VVARSMGGKEDLIWELL (Sequence No. 3)
P4 IAKIMNGEADAMSL (Sequence No. 4)
P5 YEKYLGEEYVKAV (Sequence No. 5)
P6 SKLSMGSGLNLSEPN (Sequence No. 6)

**Table 2:**

| Hybridoma (Antibody) no. | OD at 490nm (Absorbance) | | | | | |
|---|---|---|---|---|---|---|
| | P1 | P2 | P3 | P4 | P5 | P6 |
| NCDT077 | 0.026 | 0.024 | 0.023 | 0.013 | 0.018 | 0.015 |
| NCDT503 | 0.024 | 0.027 | 0.027 | 0.017 | 0.020 | 0.019 |

According to Table 2, each monoclonal antibody (Hybridoma NCDT077 producing antibody and Hybridoma NCDT503 producing antibody) does not recognize P1-P6 (peptides containing antibody recognition sites in Patent Document 3 and Patent Document 4).

Subsequently, in order to examine antigen recognition sites of antibodies prepared in the above Example 5, reactivity of the primary structure of an antigen protein was first confirmed as follows. In other words, the CDT antigen was made to covalently bind to an ELISA plate (Product name: Immobilizer Aminoantibody; manufactured by Nunc), denatured by a 4M urea, and reduced by adding 0.1M DTT to wells. An antibody adjusted to 10 µg/mL was added to each well, 50 µl per well, and made to react for 1 hour at room temperature. After washing with a wash solution (0.05% Tween-PBS), a 50 µl alkaline phosphatase labeled anti-transferrin polyclonal antibody (same as that prepared in the above Example 3) is then added and made to react for 1 hour at room temperature. After washing again, a coloration of alkaline phosphatase activity was generated using the Kind-King method. The absorbance at 490nm was measured with a microplate reader and a recognition of primary structure of protein confirmed. These results are shown below in Table 3.

**Table 3:**

| Hybridoma (Antibody) no. | OD at 490nm (Absorbance) | |
|---|---|---|
| | Untreated CDT antigen | Urea · reduction treated CDT antigen |
| NCDT077 | 3.693 | 0.132 |
| NCDT503 | 3.484 | 0.038 |

As shown in Table 3, a Hybridoma NCDT077 producing antibody and a Hybridoma NCDT503 producing antibody do not react with the primary structure of a CDT antigen that has been denatured and reduced. In addition, these antibodies do not recognize the primary structure of a CDT antigen, but recognize the conformation.

### Example 10: Confirmation of epitope discrepancy recognized by each monoclonal antibody

In order to examine the difference in the antigen recognition site of the monoclonal antibody (Hybridoma NCDT077 producing antibody and Hybridoma NCDT503 producing antibody) prepared in the above Example 5, a sandwich ELISA combined with each antibody was used. The first antibody adjusted to 10 g/mL with PBS was added to a 96-well microplate, 50µl per well, and made to react all day and night at 4°C. The wells were then washed once with PBS and blocked with 0.5% BSA-PBS. Serum of an alcoholic patient was diluted by 50-fold and 50µl of this was added to the wells and made to react for 1 hour at room temperature. After washing with a wash solution (0.05% Tween-PBS), 50µl of the other antibody labeled with biotin was added and made to react for 1 hour at room temperature. After washing again, a 50µl alkaline phosphatase labeled streptavidin was added and made to react for 30 min. at room temperature. After washing, a coloration of alkaline phosphatase activity was generated using the Kind-King method. The optical density (OD) at 490nm was measured with a microplate reader and the results are shown below in Table 4.

**Table4:**

| | | Antibody immobilized on solid phase | |
|---|---|---|---|
| | | NCDT 077 | NCDT 503 |
| Antibody for detection | NCDT 077 | 0.069 | 1.504 |
| | NCDT 503 | 1.882 | 0.152 |

According to reaction results of monoclonal antibody binding patterns shown in Table4, binding of same monoclonal antibodies showed no reaction while binding of different monoclonal antibodies showed a reaction.

### Example 11: Preparing of latex reagent 1 and 2

A 100 µl of 10% latex solution with a 0.13 µm particle size was dispensed in a 1.5 mL centrifugal tube. An 825 µl sensitization buffer solution was then added for dilution. A 75 µl anti-CDT monoclonal antibody No. 1 (Hybridoma NCDT503 producing antibody) adjusted to 10 mg/mL was added and quickly agitated and then mixed by inverting the tube in a cold place for 2 hours to conduct sensitization. After sensitization, a 100 µl blocking buffer solution was added, sonication performed for 2 min. to distribute the latex, and reaction made to occur for 1 hour at 37 °C for blocking. After completion of the blocking, the tube is centrifuged to remove the unreacted portion in the supernatant and to add a 1 mL stored buffer solution to the precipitate. Sonication is performed to distribute the latex and dilution is done for an absorbance at 700 nm to be 0.8 Abs. Adjustment of Latex reagent 1 was thus carried out. A latex reagent 2 with a sensitized anti-CDT monoclonal antibody No. 2 (Hybridoma NCDT077 producing antibody) was prepared by the same procedure.

### <Composition of sensitization buffer solution>

10 mM TES, pH 7. 5
150mM Sodium chloride
0.095% w/w Sodium azide

### <Composition of blocking buffer solution>

10 mM TES, pH 7.5
10% Bovine serum albumin
0.095% w/w Sodium azide

### <Composition of stored buffer solution>

10 mM Tris, pH 8.0
20% w/w glycerol
0.1% w/w Bovine serum albumin
0..095% w/w Sodium azide

### Example 12: Reactivity of latex reagent

The Hitachi automated analyzer Model 7170, applicable for measurements by the reagent series composed of Reagent 1, Reagent 2, and Reagent 3, was used for measurement. A buffer solution with the following composition was used as Reagent 1. The above latex reagent 1 and 2 prepared in Example 11 were used as Reagent 2 and 3 respectively. They were measured under the following 3 conditions.

In terms of specific operational procedure, a 5 µl specimen was dispensed in the reaction vessel of the automated analyzer. A 100 µ I Reagent 1 of the composition below was then dispensed. Finally, a 50 µ l Reagent 2 was dispensed for CDT in the specimen to react with latex reagent of Reagent 2. Later, the absorbance was increased when a 50 µI Reagent 3 was dispensed because of an aggregate growth of CDT and a latex reagent. The amount of change in absorbance was measured from the point when Reagent 3 was added.

In Condition A below, Latex reagent 1 and Latex reagent 2 were mixed equally to make Reagent 3 without using Reagent 2 and a 100 µl Reagent 3 was added. Reagent 1 and the measurement sample were the same and the output was obtained by an absorbance using the K-factor. Results are included in Fig. 4. It shows the relationship of the absorbance with CDT concentration. According to Fig. 4., among the antibody binding patterns used, Condition B below produces measurements with the highest sensitivity.

### <Composition of Reagent 1>

10 mM TES, pH 7.5
150mM Sodium chloride
0.095% w/w Sodium azide

### < Measurement sample>

CDT fraction, purified from human serum through ion an exchange column, diluted by saline to form 0, 3, 6, 12 and 18 mg/dl solution.

### < Measuring condition >

### A) Mixing 2 types of latex reagents

| | |
|---|---|
| Specimen | 5 µI |
| Reagent 1 | 100 µl |
| Reagent 2 | 0 µl |
| Reagent 3 | 100 µl (Latex reagent 1 and 2 mixed in equal parts) |

Measuring wavelength 570 nm dominant wavelength, 800 nm complimentary wavelength

### Photometric point 19-34

B) Allowing Latex reagent 1 to react first followed by Latex reagent 2

| | |
|---|---|
| Specimen | 5 µl |
| Reagent 1 | 100 µl |
| Reagent 2 | 50 µI (Latex reagent 1) |
| Reagent 3 | 50 µl (Latex reagent 2) |

Measuring wavelength 570 nm dominant wavelength, 800 nm complimentary wavelength

### Photometric point 19-34

C) Allowing Latex reagent 2 to react first followed by Latex reagent 1

| | |
|---|---|
| Specimen | 5 µI |
| Reagent 1 | 100 µl |
| Reagent 2 | 50 µl (Latex reagent 2) |
| Reagent 3 | 50 µl (Latex reagent 1) |

Measuring wavelength 570 nm dominant wavelength, 800 nm complimentary wavelength

### Photometric point 19-34

### Example 13: Range of latex particle size that can be used

The same sensitization method of the above Example 11 was used to sensitize an anti-CDT mouse monoclonal antibody No. 2 (Hybridoma NCDT077 producing antibody) on latex with particle size 0.09 µm, 0.13 µm, 0.15 µm, 0.20 µm, 0.34 µm, and 0.46 µm to prepare the latex reagent to have various particle sizes. The same dilution factor used for the 0.13 µm particle size in the above Example 11, was applied to all particle sizes of the reagent. The measurement sample was 0, 5, 10, 15, 20, 25, 30, 35, and 40 mg/dl CDT solution diluted with saline, and latex reagent prepared to have various particle sizes was measured under Condition B of Example 12 above. Results are included in the graph in Fig. 5. It shows the relationship of the absorbance with a CDT concentration. According to Fig. 5, an increase in the CDT dependent absorbance was confirmed for particle sizes 0.09 µm to 0.46 µm, indicating that this range of particle size can be used.

### Example 14: Range of latex concentration that can be used

Variation in concentration of Latex reagent 2 prepared in the above Example 11 were made so that the latex concentration in the reaction solution was 0.01%, 0.02%, 0.04%, 0.07%, 0.13%, 0.17%, 0.25%, and 0.5% to measure the absorbance under Condition B of Example 12 above as well as using the measurement sample from Example 13 above. Results are included in the graph in Fig. 6. It shows the relationship of the absorbance with a CDT concentration. Fig. 6 describes the sensitivity when the latex concentration in the reaction solution is changed. According to Fig. 6, sensitivity was inadequate with the 0.01% latex concentration and absorbance decreased with 0.5%. However, the increase in the CDT dependent absorbance was confirmed for 0.02% to 0.25%, indicating that this range can be used. The decrease in absorbance with 0.5% may be due to the dominant wavelength peaking out at 0 concentration because of a strong aggregation and the complimentary wavelength increasing in response to the concentration.

### Example 15: Range of monoclonal antibody concentration that can be used

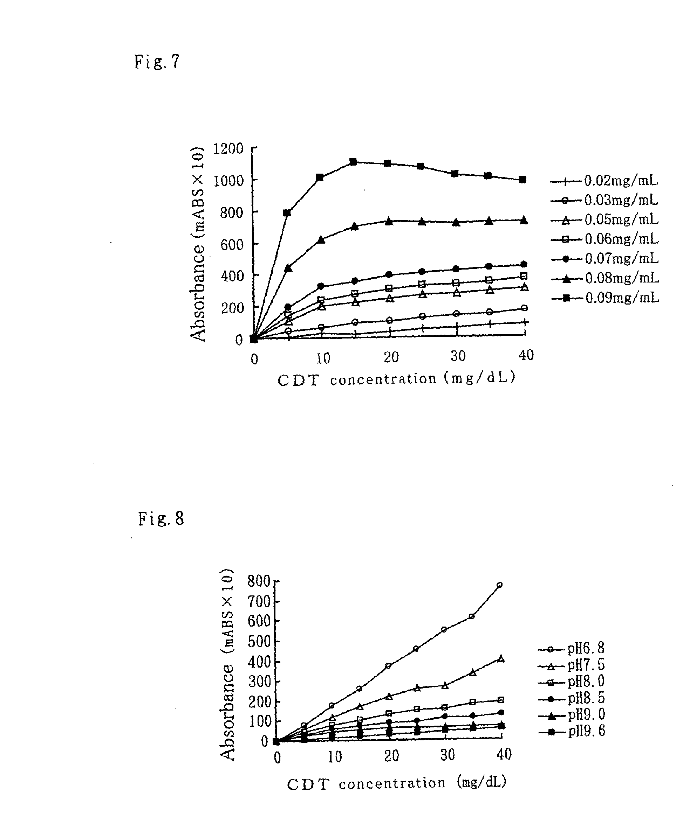

The same sensitization method of the above Example 11 was used in varying the sensitization concentration of an anti-CDT mouse monoclonal antibody No. 2 (Hybridoma NCDT077 producing antibody) to measure the absorbance under Condition B of Example 12 above as well as using the measurement sample from Example 13 above. Antibody concentrations in the solution were 0.02 mg/mL, 0.03 mg/mL, 0.05 mg/mL, 0.06 mg/mL, 0.07 mg/mL, 0.08 mg/mL, and 0.09 mg/mL. Results are included in the graph in Fig. 7. It shows the relationship of the absorbance with a CDT concentration. According to Fig. 7, sensitivity was inadequate with a 0.02 mg/mL antibody concentration in the reaction solution, but an increase in CDT-dependent absorbance was confirmed for 0.03 mg/mL to 0.09 mg/mL, indicating that this range can be used.

### Example 16: Range of pH that can be used

In addition to the Reagent 1 prepared in the above Example 12, a buffer solution with a different pH was adjusted to measure the absorbance and pH in the reaction solution under Condition B of Example 12 above as well as using the measurement sample from Example 13 above. Results are included in the graph in Fig. 8. It shows the relationship of the absorbance with a CDT concentration. According to Fig. 8, an abnormal blank reaction was observed at pH 6.5 and below. Sensitivity was inadequate at pH 9.6, but increase in a CDT-dependent absorbance was confirmed from pH 6.8 to pH 8.5, indicating that this pH range can be used.

### Example 17: Performance of measuring reagent

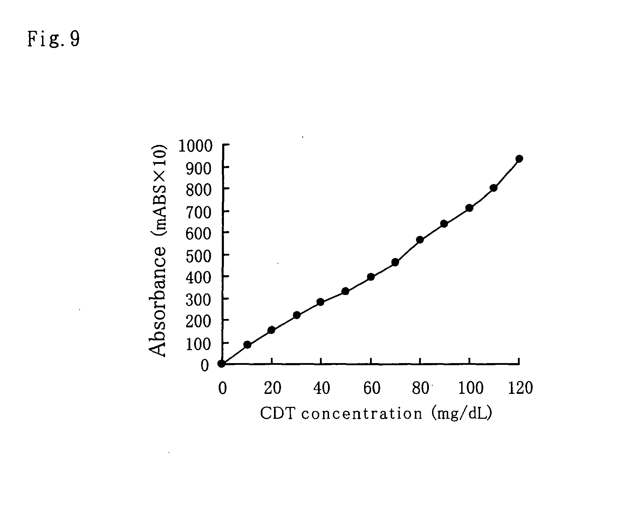

The absorbance of saline consecutively measured 20 times under Condition B of Example 12 above was converted from a 5 mg/dl CDT solution to a CDT concentration. The lower detection limit was calculated from an average + 3 SD of the 0 concentration and the results shown in Table 5. Within-run reproducibility for the 4 sample concentrations measured 10 times is shown in Table 6. The antigen overage of up to 120 mg/dl from purified a CDT fraction was measured and results included in the graph in Fig. 9. It shows the relationship of the absorbance with a CDT concentration. The lower detection limit calculated by this way is sufficiently less than a CDT concentration in the specimen of a presumably healthy person. In terms of within-run reproducibility and measuring range, the measuring reagent is considered to have adequate performance.

**Table 5:**

| No. | Measurement value of Physiological saline |
|---|---|
| 1 | 0.0 |
| 2 | -0.1 |
| 3 | -0.4 |
| 4 | 0.0 |
| 5 | 0.4 |
| 6 | 0.2 |
| 7 | -0.1 |
| 8 | 0.1 |
| 9 | -0.2 |
| 10 | -0.1 |
| 11 | 0.4 |
| 12 | 0.2 |
| 13 | -0.2 |
| 14 | -0.4 |
| 15 | 0.1 |
| 16 | -0.5 |
| 17 | -0.1 |
| 18 | -0.1 |
| 19 | 0.2 |
| 20 | -0.4 |
| Average | -0.05 |
| SD | 0.25 |
| Average + 3 SD | 0.715 |

**Table 6:**

| | Sample 1 | Sample 2 | Sample 3 | Sample 4 |
|---|---|---|---|---|
| 1 | 1.6 | 6.3 | 15.6 | 27.6 |
| 2 | 1.5 | 6.5 | 15.8 | 27.9 |
| 3 | 1.9 | 6.1 | 15.6 | 27.9 |
| 4 | 1.6 | 6.2 | 15.6 | 27.6 |
| 5 | 1.6 | 6.2 | 15.8 | 27.8 |
| 6 | 1.7 | 6.1 | 15.3 | 27.8 |
| 7 | 1.6 | 6.2 | 15.4 | 27.6 |
| 8 | 1.5 | 6.2 | 15.0 | 27.8 |
| 10 | 1.5 | 6.1 | 15.1 | 27.9 |
| Average | 1.6 | 6.22 | 15.48 | 27.75 |
| SD | 0.125 | 0.123 | 0.274 | 0.135 |
| CV | 7.8% | 2.0% | 1.8% | 0.5% |

### INDUSTRIAL APPLICABILITY:

Even in cases of poor reaction efficiency with the antibody due to steric hindrance caused by epitopes being close to each other, this invention allows efficient measurement of the desired substance. Pretreatment of the specimen is not required because specific reactions can take place simultaneously. It can therefore be used as a simple and highly sensitive immunoassay method. The measuring method of the invention is particularly suitable for measuring CDT.

## Claims

1. An immune agglutination reagent kit **characterized in that,**
comprising two types of reagents produced by separate sensitization of two types of monoclonal antibodies that recognize different epitopes on carrier particles,
in said immune agglutination reagent kit the agglutination reaction being weak when the two types of sensitized carrier particles are made to react simultaneously with a specimen containing the target antigen, and the agglutination reaction being intensifying when the order of the reaction of sensitized carrier particles is tailored for a specific type of monoclonal antibody,
said two types of reagents being independently preserved.

2. An immune agglutination reagent kit of claim 1, wherein said carrier particles are latex particles.

3. An immune agglutination reagent kit of claim 1, wherein the particle size of said carrier particle is more than 0.05 µm and less than 1 µm.

4. An immune agglutination reagent kit of claim 1, wherein the antigen recognized by said monoclonal antibodies is CDT.

5. An immune agglutination reagent kit of claim 1, wherein one specific type of monoclonal antibody is anti-CDT mouse monoclonal antibody produced by Hybridoma NCDT503 (FERM P-20844) sensitized on carrier particles as a first reagent, and the other specific type of monoclonal antibody is anti-CDT mouse monoclonal antibody produced by Hybridoma NCDT077 (FERM P-20843) sensitized on carrier particles as a second reagent.

6. An immune agglutination reagent kit of claim 1, wherein said carrier particles are latex particles.

7. A method of measuring antigen comprising the step of:
preparing the immune agglutination reagent kit according to any one of claims 1-5,
one type of reagent being made to react with antigen in specimen suspected to contain antigen,
followed by the other type of reagent being made to react with the obtained reaction solution to generate agglutination,
measuring the generated amount of change in agglutination.

8. A method of measuring antigen of claim 7, wherein using of an automated analyzer or a manual method.

9. A method of measuring antigen of claim 7, wherein the carrier particle concentration of said reaction solution is more than 0.02% w/w and less than 0.25% w/w.

10. A method of measuring antigen of claim 7, wherein the monoclonal antibody concentration of said reaction solution is more than 0.03 mg/mL and less than 0.09 mg/mL.

11. A method of measuring antigen of claim 7, wherein the pH of said reaction solution is more than 6.8 and less than 8.5.

12. A method of measuring antigen of claim 7, wherein said antigen is CDT.

13. A method of measuring antigen comprising the step of:
reagent containing anti-CDT mouse monoclonal antibodies No. 1 produced by Hybridoma NCDT503 (FERM P-20844) sensitized with carrier particles being made to react with antigen in specimen suspected to contain CDT,
followed by reagent containing anti-CDT mouse monoclonal antibodies produced by Hybridoma NCDT077 (FERM P-20843) sensitized with carrier particles being made to react with the obtained reaction solution to generate agglutination,
measuring the generated amount of change in agglutination.

14. A Method of measuring antigen of claim 7 or 13, wherein said amount of change in agglutination being the amount of change in absorbance.

15. An Anti-CDT mouse monoclonal antibody produced by Hybridoma NCDT077 (FERM P-20843) used in the method of measuring antigen of claim 13.

16. An Anti-CDT mouse monoclonal antibody of claim 15, wherein said monoclonal antibodies have no reactivity against the following P1-P6 peptide domains or have virtually no reactivity:
P1: VLAENYNKSDNCE
P2: QHLFGSNVTDCSG
P3: WARSMGGKEDLIWELL
P4: IAKIMNGEADAMSL
P5: YEKYLGEEYVKAV
P6: SKLSMGSGLNLSEPN.

17. Hybridoma NCDT077 (FERM P-20843).

## Amended claims

### Amended claims under Art. 19.1 PCT

**1.** (amended) An immune agglutination reagent kit for measuring CDT **characterized in that,**
comprising two types of reagents produced by separate sensitization of two types of anti-CDT monoclonal antibodies that recognize different epitopes on carrier particles,
in said immune agglutination reagent kit the agglutination reaction being weak when the two types of sensitized carrier particles are made to react simultaneously with a specimen containing the target CDT antigen, and the agglutination reaction being intensifying when the order of the reaction of sensitized carrier particles is tailored for a specific type of anti-CDT monoclonal antibody,
said two types of reagents being independently preserved.

**2.** (delete)

**3.** (delete)

**4.** (delete)

**5.** An immune agglutination reagent kit of cliam 1, wherein one specific type of monoclonal antibody is anti-CDT mouse monoclonal antibody produced by Hybridoma NCDT503 (FERM P-20844) sensitized on carrier particles as a first reagent, and the other specific type of monoclonal antibody is anti-CDT mouse monoclonal antibody produced by Hybridoma NCDT077 (FERM P-20843) sensitized on carrier particles as a second reagent.

**6.** (delete)

**7.** (delete)

**8.** (delete)

**9.** (delete)

**10.** (delete)

**11.** (delete)

**12.** (delete)

**13.** (delete)

**14.** (delete)

**15.** (delete)

**16.** (amended) An anti-CDT mouse monoclonal antibody used in the immune agglutination reagent kit of cliam 5, wherein Anti-CDT mouse monoclonal antibody produced by Hybridoma NCDT077 (FERM P-20843) have no reactivity against the following P1-P6 peptide domains or have virtually no reactivity:
P1: VLAENYNKSDNCE
P2: QHLFGSNVTDCSG
P3: VVARSMGGKEDLIWELL
P4: IAKIMNGEADAMSL
P5: YEKYLGEEYVKAV
P6: SKLSMGSGLNLSEPN.
